# EUROPEAN PATENT SPECIFICATION

(11) **EP 4 326 370 B1**
(45) Date of publication and mention of the grant of the patent: **19.11.2025**
(21) Application number: 22724461.3
(22) Date of filing: 22.04.2022
(51) Int. Cl.: A61M 5/315, A61M 5/20

(54) **ELECTRONIC SYSTEM FOR A DRUG DELIVERY DEVICE**
ELEKTRONISCHES SYSTEM FÜR EINE WIRKSTOFFFREISETZUNGSVORRICHTUNG
SYSTÈME ÉLECTRONIQUE POUR DISPOSITIF D'ADMINISTRATION DE MÉDICAMENTS

(30) Priority: 23.04.2021 EP 21315069; 10.01.2022 EP 22315005
(43) Date of publication of application: 28.02.2024
(73) Proprietor: Sanofi, 75017 Paris (FR)
(72) Inventor: ALLERDINGS, Alexander, 65926 Frankfurt am Main (DE); HARVEY-COOK, Adam, Moyo, Essex, CM12 OUD (GB); JONES, Matthew, Meredith, Warwick, CV34 4AB (GB); O'HARE, Aidan, Michael, Warwick, CV34 4AB (GB)
(74) Representative: Keil & Schaafhausen Patentanwälte PartGmbB
(86) International application number: PCT/EP2022/060641
(87) International publication number: WO 2022/223747

(56) References cited:
- WO-A1-2019/101962
- WO-A1-2019/101962
- WO-A1-2021/116387
- WO-A1-2021/116387

## Description

The present invention is generally directed to an electronic system for a drug delivery device. The present invention further relates to a drug delivery device, which preferably comprises the electronic system. In more detail, the present invention relates to features of an electronic module that can be embodied as a re-usable clip-on module with a suitably configured pen injector for the purpose of recording doses that are delivered from it.

Pen type drug delivery devices have application where regular injection by persons without formal medical training occurs. This may be increasingly common among patients having diabetes where self-treatment enables such patients to conduct effective management of their disease. In practice, such a drug delivery device allows a user to individually select and dispense a number of user variable doses of a medicament.

There are basically two types of drug delivery devices: resettable devices (i.e., reusable) and non-resettable (i.e., disposable). For example, disposable pen delivery devices are supplied as self-contained devices. Such self-contained devices do not have removable pre-filled cartridges. Rather, the pre-filled cartridges may not be removed and replaced from these devices without destroying the device itself. Consequently, such disposable devices need not have a resettable dose setting mechanism. The present invention is applicable for disposable and reusable devices.

For such devices the functionality of recording doses that are dialled and delivered from the pen may be of value to a wide variety of device users as a memory aid or to support detailed logging of dose history. Thus, drug delivery devices using electronics are becoming increasingly popular in the pharmaceutical industry as well as for users or patients. For example, a drug delivery device is known from EP 2 729 202 B1 comprising an electronically controlled capturing system for capturing data related to the amount of drug expelled from a reservoir by expelling means.

However, especially if the device is designed to be self-contained, that is to say without a connector for a connection to an electrical power source which is necessary to provide electrical power for the operation of the device, the management of the resources of a power supply integrated into the device is particularly important. WO 2021/191327 A1 addresses the problem of power consumption when the device is not in use and proposes different power consumption states for extending battery life.

The prior art regarding switching between power consumption states includes several alternatives. For example, EP 3 525 844 B1 proposes a monitoring unit including a light sensor and a touch sensor, wherein the touch sensor is operably connected to the electronics circuit such that the electronics circuit is activated when the touch sensor detects a touch or a movement of the monitoring unit. This may save power of the monitoring device in that it can be put in inactive or sleep mode when not used and may be activated when a user picks up a medicament delivery device having the monitoring unit attached. Further, WO 2013/004844 A1 proposes an actuation member adapted to be moved into an intermediate position in which a switch means is actuated to start initialization of a capturing system.

EP 2 767 297 B1 discloses a diabetes management system that includes a drug delivery pen configured to wirelessly communicate with a data management unit. The pen may include a dosage sensor, e.g. an optical sensor, to monitor movement of a knob for monitoring the activity of the drug delivery pen. Further, an inertial sensor may also be used to wake up the device if it is in a sleep mode when the device is picked up by the user.

Additional examples of motion detections sensors used in monitoring units for drug delivery devices include accelerometers as disclosed in EP 2 911 717 B1 for detecting an auditory signal, in EP 2 926 846 B1 for determining if a pen was primed properly, whether insulin types were mixed properly in pen, or if a pen should come out of sleep mode. Further accelometers used in drug delivery devices are known e.g. from EP 2 401 006 B1, WO 2016/071912 A1 and WO 2017/189970 A1.

In WO 2019/219825 A1, a drug delivery assembly comprises an electronic sensor circuitry with a light-based non-contact tracking means adapted to determine a motion property of a surface moved relative to the tracking means. This electronic sensor circuitry can be operated between a sleep state, a low power detection state allowing a first motion property to be determined, and a high-power measuring state allowing a second motion property to be determined. The electronic sensor circuitry is adapted to change the operative state from the sleep state to the detection state and back to the sleep state with pre-determined intervals. Detecting a motion by means of a light-based sensor requires detection over a certain period. In addition, the intervals must be short enough to detect even a quick dose setting action. Both results in a relatively high power consumption. However, a further reduction of the power consumption may be desirable during a long-term condition of not using the sensor circuitry, e.g. during storage of the device prior to the first use.

WO 2019/101962 A1 and WO 2021/116387 A1 diesclose modules for drug delivery devices having a low power state in which optical sensors are not energized and a high power state in which optical sensors are energized. The systems may be switched into the high power state by a switch activated by the displacement of an injection button.

Thus, it is an object of the present disclosure to provide improvements regarding energy management for drug delivery devices comprising an electronic system or electronic systems for drug delivery devices.

This object is solved by the subject matter defined in the independent claim.

Advantageous embodiments and refinements are subject to the dependent claims.

One aspect of the disclosure relates to an electronic system for a drug delivery device. Another aspect of the disclosure relates to a drug delivery device comprising such an electronic system. Accordingly, the features described herein with relation to the drug delivery device should be considered as being disclosed for the electronic system and vice versa.

An electronic system for use with a drug delivery device may be suitable for recording doses that are delivered from the drug delivery device. The electronic system may comprise an electrical power supply, e.g. a battery, like a coin cell type battery, a memory for storing data, a processor configured to control operation of the electronic system and coupled to the electrical power supply and to the memory. In addition, the electronic system may comprise at least two optical sensor units, e.g. a first light source with a corresponding first optical sensor and a second light source with a corresponding second optical sensor, which are in communication with the processor. The optical sensors may be suitable for detecting a movement of an encoder of the drug delivery device wherein the movement is indicative of doses that are dialed (i.e. selected) and/or delivered from the drug delivery device. To reduce the power consumption, the electronic system may have a first low-power-consumption state and at least one further state having a higher power consumption compared with the first state.

There are several different ways suitable to implement the optical sensor units. For example, the optical sensor unit(s) may comprise a radiation detector comprising an electromagnetic radiation emitter, e.g. an LED, like in IR-LED, and a radiation detector.

In one example, the encoder and the optical sensor units are in an anti-phase arrangement which means that if both light sources simultaneously emit light, only one of the optical sensors receives the light whereas the other optical sensor does not receive the emitted light. As the encoder and the sensor units are moved relative to each other, the optical sensor which previously received the light now does not receive the emitted light whereas the other optical sensor does receive the light. This may be achieved by the encoder selectively reflecting light. As an alternative, the encoder may selectively block light. In other examples the encoder and the optical sensor units are not in an anti-phase arrangement, such that if both light sources simultaneously emit light, none or only one or all optical sensors detect the light depending on the relative position of the encoder.

The electronic system may be configured as a re-usable clip-on module for an injection device. As an alternative, the electronic system may be a unit or module integrated (built in) into an injection device. The terms electronic system and (electronic) module are used synonymously in the following for both alternatives. The functionality of recording doses may be of value to a wide variety of device users as a memory aid or to support detailed logging of dose history. It is envisaged that the electronic system, e.g. an electronic module, could be configured to be connectable to a mobile phone, or similar, to enable the dose history to be downloaded from the system on a periodic basis.

According to the present disclosure, at least one of the optical sensor units may be used to for waking the electronic encoding module from the first low-power-consumption state to the at least one further state. Storing the electronic module in a low-power state with a means of waking into a higher-power state, e.g. for dose detection, storage and transmission, limits power consumption when the device is not in use and extends battery life. Using optical sensors already present in the electronic module (e.g. for dose encoding) to perform this function obviates the need for any additional hardware specifically to achieve this aim. There are a number of different examples of such an optical sensing system, as described below.

In a first aspect of the present disclosure the processor may be configured to regularly poll both optical sensors in the first low-power-consumption state, i.e. a light signal is emitted by the light sources and it is detected wether or not the light is received by the optical sensors. In contrast to a motion detection by means of optical sensors, a single light flash is sufficient for polling the optical sensors. This reduces power consumption. For example, the processor may be configured to maintain the first low-power-consumption state if the response of both optical sensors is identical to each other and identical to the preceding response and to switch into the at least one further state if the responses of the optical sensors are different from each other and/or are different from the preceding response.

For example, in an electronic system for use with a drug delivery device for recording doses that are delivered from the drug delivery device, the electronic system comprises: an electrical power supply; a memory; a processor configured to control operation of the electronic system and coupled to the electrical power supply and to the memory; and a first light source, a second light source, a first optical sensor and a second optical sensor which are in communication with the processor, wherein the optical sensors are suitable for detecting a movement of an encoder (arranged and configured such that the movement is indicative of doses that are dialed and/or delivered from the drug delivery device) of the drug delivery device in an anti-phase arrangement (which means that if both light sources simultaneously emit light, only one of the optical sensors receives the light whereas the other optical sensor does not receive the emitted light) which movement is indicative of doses that are dialed and/or delivered from the drug delivery device, the electronic system having a first low-power-consumption state and at least one further state having a higher power consumption compared with the first state. In such an electronic system, the processor may be configured to regularly poll both optical sensors (wherein polling the sensors is achieved by a light signal being emitted by the light sources and it is detected wether or not the light is received by the respective optical sensors) in the first low-power-consumption state, to maintain the first low-power-consumption state if the response of both optical sensors is identical to each other and identical to the preceding response, and to switch into the at least one further state if the responses of the optical sensors are different from each other and/or are different from the preceding response.

In an electronic system with the encoder and the optical sensor units being in an anti-phase arrangement, if during polling the response of both optical sensors is identical to each other, this may be an indication that the electronic system is not in an arrangement suitable for dose detection. Such an arrangement may occur for example if the electronic system is stored prior to the first use remote from the drug delivery device. As an alternative, this arrangement may occur if the electronic system is mounted on a drug delivery device, but the sensors are too far away from the encoder, e.g. in an initial position prior to the first use of the drug delivery device. In both alternative, it is desirable to keep power consumption as low as possible.

In more detail, the first light source and the first optical sensor may be arranged next to each other and facing towards a sensor side end of a first light pipe, wherein the second light source and the second optical sensor may be arranged next to each other and facing towards a sensor side end of a second light pipe. Further, the light pipes may each have an opposite encoder side end which if occluded by a light reflective material result in a light beam emitted from the respective first or second light source to be detected by the respective first or second optical sensor. For example, the electronic system may further comprise at least one removable occluding element positioned in the respective light path between the light sources and the respective optical sensors. Still further, the removable occluding element may comprise a reflective surface for occluding the optical sensors.

The electronic system or module may be stored in a low-power long-term storage mode when placed in its original packaging immediately after manufacture to reduce battery discharge during long-term storage prior to its first use. In this low-power long-term storage mode, one of the mechanisms for reducing power consumption of the electronic circuit is to disable detection of user inputs. The act of removing the module from its packaging prior to its first use may wake it from this storage mode into a higher-power mode capable of detecting user inputs.

More specifically, a packaging of the electronic system may include a reflective surface which occludes the end faces of two light pipes connected to two optical sensors in the module. The optical sensors are polled regularly but infrequently during the low-power long-term storage mode. For example, the processor may be configured to regularly poll the optical sensors in the first low-power-consumption state, for example every 10 to 40 seconds, ideally every 15 to 25 seconds, e.g. every 20 seconds, wherein the processor is configured to wake the electronic system, e.g. to regularly poll the optical sensors in the at least one further state with a higher frequency than in the first low-power-consumption state. The processor may be configured to regularly poll the optical sensors in the first low-power-consumption state every 5 to 15 seconds or every 15 to 25 seconds or every 25 to 50 seconds. If when the optical sensors are polled both remain occluded, the module may remain in the low power long-term storage mode. In other words, the processor may be configured to maintain the first low-power-consumption state if the response of both optical sensors is that light beams emitted from both light source are detected by the respective first and second optical sensors. This polling may be used only prior to first use; once it has been detected using this method that the module has been removed from the packaging, this mode of regular polling may cease.

When the module is removed from the packaging, but before it is fitted to an injection device, neither optical sensor is occluded. Likewise, when the module is fitted to an injection device requiring approximation of the sensors to the encoder by pressing a dose button, with the dose button not being depressed neither optical sensor is occluded. If when the sensors are polled, the module is in this neither-occluded state, the module may wake. When the module is fitted to such an injection device and the dose button is depressed (e.g. to pair the module to a mobile phone using Bluetooth), one single optical sensor is occluded by the encoder of the injection pen. If the module is rapidly removed from its packaging and fitted to an injection device and the user holds the dose button in, such that the sensors are not polled in the neither-occluded state described above, when they are polled in this single-occluded state the module may wake.

The electronic system may further comprise a communication unit for communicating with another device. Preferably, the electronic system is configured such that it is switched from the first state into the second state, thereby inducing the communication unit to establish said communication with another device, e.g. a syncronisation or pairing operation. The electronic control unit may issue a command, e.g. a signal, to another unit of the electronic system such that this unit is switched on or rendered operational. This unit may be the communication unit for communicating with another device, e.g. a wireless communications interface for communicating with another device via a wireless network such as Wi-Fi or Bluetooth, or even an interface for a wired communications link, such as a socket for receiving a Universal Series Bus (USB), mini-USB or micro-USB connector. Preferably, the electronic system comprises an RF, WiFi and/or Bluetooth unit as the communication unit. The communication unit may be provided as a communication interface between the system or the drug delivery device and the exterior, such as other electronic devices, e.g. mobile phones, personal computers, laptops and so on. For example, dose data may be transmitted by the communication unit to the external device. The dose data may be used for a dose log or dose history established in the external device.

According to a further aspect of the present disclosure, the processor may be configured to regularly poll the optical sensors in the first low-power-consumption state for example every 0.5 to 2 seconds, e.g. with a frequency of 1 Hz, and may be configured to wake further components of the electronic system the at least one further state. The frequency may be between 0.2 Hz and 0.7 Hz or between 0,7 Hz and 1.3 Hz or between 1.3 Hz and 3 Hz or between 3 Hz and 5 Hz. For example, the processor may be configured to regularly poll the optical sensors in with a higher frequency than in the first low-power-consumption state and/or to switch on (i.e. wake) the communication unit. For example, the processor may be configured to maintain the first low-power-consumption state if the response of both optical sensors is that light beams emitted from both light sources are detected by neither of both optical sensors and to switch into the at least one further state if the responses of the optical sensors are different from each other and/or are different from the preceding response.

The constant low-frequency polling of e.g. 1 Hz of a pair of optical sensors in the electronic system or module may be used in low-power mode for example to detect when the user has depressed the injection device dose button in order to syncronize or pair the module with a mobile phone using Bluetooth. This constant low-frequency polling may be used in low-power mode following first use when the module is inactive, i.e. a dose is not being recorded. It may also be used while the module is in the packaging, but before it is fitted to an injection device.

In more detail, when the module is not attached to an injection device (e.g. it has not yet been removed from its original packaging), the optical sensors may be in the neither-occluded state if no reflective occluding element is provided. Likewise when the module has been fitted to an injection device but the dose button has not been depressed (e.g. the module has not been paired with a mobile phone using Bluetooth), the optical sensors may be in the neither-occluded state. If the sensors are polled in this neither-occluded state, the module will not wake. However, when the module is fitted to an injection device and the dose button is depressed, one of the two optical sensors may be occluded in the anti-phase arrangement. If the user holds the dose button in this state for a long enough period (e.g. several seconds to initiate pairing) the optical sensors will be polled in this single-occluded state. This will cause the module to wake.

According to a further aspect of the present disclosure, the processor may be configured to regularly poll the optical sensors in the first low-power-consumption state with a frequency of, for example 100 Hz to 300 Hz, e.g. 200 Hz, and the processor may be configured to wake further components of the electronic system, to initiate additional funcions and/or to regularly poll the optical sensors in the at least one further state with a higher frequency than in the first low-power-consumption state. The frequency may be between 50 Hz and 150 Hz or between 150 Hz and 250 Hz or between 250 Hz and 400 Hz. In more detail, the at least one further state of the electronic system may be a dose recording state in which, when the electronic system is attached to or integrated into a drug delivery device, the light sources and the optical sensors are used to detect dose dialing and/or dose delivering. The processor may be configured to process and/or record the responses of the optical sensors in the dose recording state.

In this example, constant moderate-frequency polling (e.g. 200Hz) of a pair of optical sensors in the module may be used in low-power mode to detect when the user has begun to dispense a dose using the injection device. This constant low-frequency polling may be used in low-power mode following first use when the module is inactive, i.e. a dose is not being recorded. It may also be used while the module is in the packaging, but before it is fitted to an injection device.

In the anti-phase arrangement, when the dose button is first pressed, one of the two optical sensors will be occluded by features on the encoder and the other will not. As the first unit of drug is dispensed, the encoder will move relative to the sensors, e.g. rotate, such that the optical sensor which was initially occluded ceases to be so, and the optical sensor which was not initially occluded becomes so. This moderate-frequency polling of the optical sensors will allow this change of occlusion state to be detected, and the module may wake and immediately record the dose being dispensed.

According to a still further aspect of the present disclosure, the electronic system further has a sleeping state in which the light sources are not activated (not provided with power from the power source). The electronic system may further comprise at least one motion sensor suitable for detecting movement of the electronic system. In this example, the processor may be configured to maintain the sleeping state if no movement is detected by the at least one motion sensor and to switch into the first low-power-consumption state or into the at least one further state if a movement is detected by the at least one motion sensor. Generally, a sleeping state or mode may be a mode in which all functionalities of the module are at minimal or virtually zero power consumption but which does not require a system boot up in the event that the electronic system (or the drug delivery device) is taken out of sleeping mode.

For example, such a motion sensor may be used while the electronic system is in its low-power mode to detect whether the module is stationary. If no movement is detected, the module is assumed to be in storage or idle, with no user interaction occurring, the optical sensors are not polled and the electronic system or module does not wake. If movement is detected, it is assumed that the user may be interacting with the module, the electronic system or module partially wakes into the low-frequency polling mode or into the moderate-frequency polling mode described above. This reduces the amount of power being drawn when the module is sitting stationary, and increases battery life.

As an example for the motion sensor, an accelerometer may used to keep the device in a low-power standby mode while the device is stationary. Accelerometers may have very low power modes of <1uA., e.g.even <300nA, such that energy consumption in the sleeping mode, i.e. a mode in which the motion sensor is active but most or all other functions are inactive, is significantly lower compared to the first low-power-consumption state having for example a quiescent current of ~320 µA when polling the sensors at 200Hz. Suitable exampes of such accelerometers include i.a. the MelexisMC3635 which has two sniff modes: 1 Hz (0.3 µA @ 1.8V) and 6 Hz (0.4 µA @ 1.8V).

In an example, if motion activates the device, it will poll the sensors at 200 Hz until a dose is detected or until a time-out brings the device back into the sleep mode, e.g. a 20 s time-out. Generally, an accelerometer is liable to erroneous activations due to shock or vibrations from the environment. This may result in a battery lifetime (e.g. for a non-rechargeable coin cell battery) of about one year if the accelerometer wakes the device for up to 200 minutes per day. For users spending a long time in motorised transport or walking, the awake time will be higher and the battery life will be reduced.

As an alternative, the power may be supplied by a rechargeable battery. This gives effectively unlimited battery life. Recharging may be achieved using a recharging station and physical contact pins. The otional use of an accelerometer helps to prolong the time between recharging events.

If the electronic system is a re-usable module for releasable attachment to a drug delivery device, the electronic system or module may comprise an outer cap with a cenral axis, a chassis which is at least partially retained within the cap and a PCB comprising the memory and the processor. For example, the PCB and the electrical power supply may be retained in the cap and the chassis. Further, the light sources and the optical sensors may be arranged on a circular region about the central axis, with the first light source and the first optical sensor being angularly offset from the second light source and the second optical sensor. For example, the chassis may be at least partially transparent and may comprise the light pipes. In other words, the light pipes may be formed as a protrusion of the transparent chassis.

According to a further aspect of the present disclosure, the light pipes are arranged between 30° and 60°, e.g. 45°, apart from each other. In order to establish an anti-phase arrangement of the sensors with the encoder, the encoder may have a pattern of reflecting elements spaced by non-reflecting areas, e.g. light absorbing areas or void spaces. For example, with the light pipes arranged 45° apart from each other, the encoder may comprise a ring of 12 light reflecting teeth spaced by 30° from each other.

A drug delivery device according to the present disclosure may comprise an electronic system as described above and a dose setting and drive mechanism which is configured to perform a dose dialing operation for selecting a dose to be delivered by the drug delivery device and a dose delivery operation for delivering the set dose. The dose setting and drive mechanism may comprise the encoder rotatable at least in the dose delivery operation and/or in the dose setting operation. Preferably, the pattern of the encoder is adapted to the location of the optical sensor units to establish an anti-pahes arrangement of the sensor units. In other words the electronic system may be arranged such that depending on the rotational position of the encoder light emitted by one of the light sources is reflected by one of the teeth and detected by the respective optical sensor, while light emitted by the other of the light sources is not reflected by one of the teeth. This means that the two optical sensors are effectively in anti-phase and for each dose unit that is dispensed, the two channels of the sensor units cross over.

In one embodiment, the drug delivery device comprises a dial sleeve, e.g. a number sleeve, or a member axially and/or rotationally locked thereto which is rotatable relative to a housing of the dose setting and drive mechanism, e.g. along a helical path, at least in the dose setting operation. In addition, a dose and/or injection button or a member axially and/or rotationally locked thereto may be axially displaceable relative to the dial sleeve and rotationally constrained to the housing at least in the dose delivery operation. According to one aspect of the disclosure, the dial sleeve comprises the encoder ring. The encoder ring may be, permanently or releasably, clipped on the dial sleeve. It may be a unitary component with the dial sleeve. The encoder ring may have a plurality of teeth extending radially outwards and spaced from each other. The distance between two adjacent teeth may correspond to one dose unit. In other words, rotation of the dial sleeve for selecting or delivering one dose unit results in rotation of the encoder ring from one tooth to the adjacent tooth.

The present disclosure is applicable for devices which are manually driven, e.g. by a user applying a force to an injection button, for devices which are driven by a spring or the like and for devices which combine these two concepts, i.e. spring assisted devices which still require a user to exert an injection force. The spring-type devices involve springs which are preloaded and springs which are loaded by the user during dose selecting. Some stored-energy devices use a combination of spring preload and additional energy provided by the user, for example during dose setting.

The present disclosure further pertains to a drug delivery device with the electronic system as described above which drug delivery device comprises a cartridge containing a medicament.

The terms "drug" or "medicament" are used synonymously herein and describe a pharmaceutical formulation containing one or more active pharmaceutical ingredients or pharmaceutically acceptable salts or solvates thereof, and optionally a pharmaceutically acceptable carrier. An active pharmaceutical ingredient ("API"), in the broadest terms, is a chemical structure that has a biological effect on humans or animals. In pharmacology, a drug or medicament is used in the treatment, cure, prevention, or diagnosis of disease or used to otherwise enhance physical or mental well-being. A drug or medicament may be used for a limited duration, or on a regular basis for chronic disorders.

As described below, a drug or medicament can include at least one API, or combinations thereof, in various types of formulations, for the treatment of one or more diseases. Examples of API may include small molecules having a molecular weight of 500 Da or less; polypeptides, peptides and proteins (e.g., hormones, growth factors, antibodies, antibody fragments, and enzymes); carbohydrates and polysaccharides; and nucleic acids, double or single stranded DNA (including naked and cDNA), RNA, antisense nucleic acids such as antisense DNA and RNA, small interfering RNA (siRNA), ribozymes, genes, and oligonucleotides. Nucleic acids may be incorporated into molecular delivery systems such as vectors, plasmids, or liposomes. Mixtures of one or more drugs are also contemplated.

The drug or medicament may be contained in a primary package or "drug container" adapted for use with a drug delivery device. The drug container may be, e.g., a cartridge, syringe, reservoir, or other solid or flexible vessel configured to provide a suitable chamber for storage (e.g., short- or long-term storage) of one or more drugs. For example, in some instances, the chamber may be designed to store a drug for at least one day (e.g., 1 to at least 30 days). In some instances, the chamber may be designed to store a drug for about 1 month to about 2 years. Storage may occur at room temperature (e.g., about 20°C), or refrigerated temperatures (e.g., from about - 4°C to about 4°C). In some instances, the drug container may be or may include a dual-chamber cartridge configured to store two or more components of the pharmaceutical formulation to-be-administered (e.g., an API and a diluent, or two different drugs) separately, one in each chamber. In such instances, the two chambers of the dual-chamber cartridge may be configured to allow mixing between the two or more components prior to and/or during dispensing into the human or animal body. For example, the two chambers may be configured such that they are in fluid communication with each other (e.g., by way of a conduit between the two chambers) and allow mixing of the two components when desired by a user prior to dispensing. Alternatively or in addition, the two chambers may be configured to allow mixing as the components are being dispensed into the human or animal body.

The drugs or medicaments contained in the drug delivery devices as described herein can be used for the treatment and/or prophylaxis of many different types of medical disorders. Examples of disorders include, e.g., diabetes mellitus or complications associated with diabetes mellitus such as diabetic retinopathy, thromboembolism disorders such as deep vein or pulmonary thromboembolism. Further examples of disorders are acute coronary syndrome (ACS), angina, myocardial infarction, cancer, macular degeneration, inflammation, hay fever, atherosclerosis and/or rheumatoid arthritis. Examples of APIs and drugs are those as described in handbooks such as Rote Liste 2014, for example, without limitation, main groups 12 (antidiabetic drugs) or 86 (oncology drugs), and Merck Index, 15th edition.

Examples of APIs for the treatment and/or prophylaxis of type 1 or type 2 diabetes mellitus or complications associated with type 1 or type 2 diabetes mellitus include an insulin, e.g., human insulin, or a human insulin analogue or derivative, a glucagon-like peptide (GLP-1), GLP-1 analogues or GLP-1 receptor agonists, or an analogue or derivative thereof, a dipeptidyl peptidase-4 (DPP4) inhibitor, or a pharmaceutically acceptable salt or solvate thereof, or any mixture thereof. As used herein, the terms "analogue" and "derivative" refers to a polypeptide which has a molecular structure which formally can be derived from the structure of a naturally occurring peptide, for example that of human insulin, by deleting and/or exchanging at least one amino acid residue occurring in the naturally occurring peptide and/or by adding at least one amino acid residue. The added and/or exchanged amino acid residue can either be codable amino acid residues or other naturally occurring residues or purely synthetic amino acid residues. Insulin analogues are also referred to as "insulin receptor ligands". In particular, the term "derivative" refers to a polypeptide which has a molecular structure which formally can be derived from the structure of a naturally occurring peptide, for example that of human insulin, in which one or more organic substituent (e.g. a fatty acid) is bound to one or more of the amino acids. Optionally, one or more amino acids occurring in the naturally occurring peptide may have been deleted and/or replaced by other amino acids, including non-codeable amino acids, or amino acids, including non-codeable, have been added to the naturally occurring peptide. Examples of insulin analogues are Gly(A21), Arg(B31), Arg(B32) human insulin (insulin glargine); Lys(B3), Glu(B29) human insulin (insulin glulisine); Lys(B28), Pro(B29) human insulin (insulin lispro); Asp(B28) human insulin (insulin aspart); human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Examples of insulin derivatives are, for example, B29-N-myristoyl-des(B30) human insulin, Lys(B29) (N- tetradecanoyl)-des(B30) human insulin (insulin detemir, Levemir^{®}); B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-gamma-glutamyl)-des(B30) human insulin, B29-N-omega-carboxypentadecanoyl-gamma-L-glutamyl-des(B30) human insulin (insulin degludec, Tresiba^{®}); B29-N-(N-lithocholyl-gamma-glutamyl)-des(B30) human insulin; B29-N-(ω-carboxyheptadecanoyl)-des(B30) human insulin and B29-N-(w-carboxyheptadecanoyl) human insulin.

Examples of GLP-1, GLP-1 analogues and GLP-1 receptor agonists are, for example, Lixisenatide (Lyxumia^{®}), Exenatide (Exendin-4, Byetta^{®}, Bydureon^{®}, a 39 amino acid peptide which is produced by the salivary glands of the Gila monster), Liraglutide (Victoza^{®}), Semaglutide, Taspoglutide, Albiglutide (Syncria^{®}), Dulaglutide (Trulicity^{®}), rExendin-4, CJC-1134-PC, PB-1023, TTP-054, Langlenatide / HM-11260C (Efpeglenatide), HM-15211, CM-3, GLP-1 Eligen, ORMD-0901, NN-9423, NN-9709, NN-9924, NN-9926, NN-9927, Nodexen, Viador-GLP-1, CVX-096, ZYOG-1, ZYD-1, GSK-2374697, DA-3091, MAR-701, MAR709, ZP-2929, ZP-3022, ZP-DI-70, TT-401 (Pegapamodtide), BHM-034. MOD-6030, CAM-2036, DA-15864, ARI-2651, ARI-2255, Tirzepatide (LY3298176), Bamadutide (SAR425899), Exenatide-XTEN and Glucagon-Xten.

An example of an oligonucleotide is, for example: mipomersen sodium (Kynamro^{®}), a cholesterol-reducing antisense therapeutic for the treatment of familial hypercholesterolemia or RG012 for the treatment of Alport syndrom.

Examples of DPP4 inhibitors are Linagliptin, Vildagliptin, Sitagliptin, Denagliptin, Saxagliptin, Berberine.

Examples of hormones include hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, and Goserelin.

Examples of polysaccharides include a glucosaminoglycane, a hyaluronic acid, a heparin, a low molecular weight heparin or an ultra-low molecular weight heparin or a derivative thereof, or a sulphated polysaccharide, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium. An example of a hyaluronic acid derivative is Hylan G-F 20 (Synvisc^{®}), a sodium hyaluronate.

The term "antibody", as used herein, refers to an immunoglobulin molecule or an antigen-binding portion thereof. Examples of antigen-binding portions of immunoglobulin molecules include F(ab) and F(ab')2 fragments, which retain the ability to bind antigen. The antibody can be polyclonal, monoclonal, recombinant, chimeric, de-immunized or humanized, fully human, non-human, (e.g., murine), or single chain antibody. In some embodiments, the antibody has effector function and can fix complement. In some embodiments, the antibody has reduced or no ability to bind an Fc receptor. For example, the antibody can be an isotype or subtype, an antibody fragment or mutant, which does not support binding to an Fc receptor, e.g., it has a mutagenized or deleted Fc receptor binding region. The term antibody also includes an antigen-binding molecule based on tetravalent bispecific tandem immunoglobulins (TBTI) and/or a dual variable region antibody-like binding protein having cross-over binding region orientation (CODV).

The terms "fragment" or "antibody fragment" refer to a polypeptide derived from an antibody polypeptide molecule (e.g., an antibody heavy and/or light chain polypeptide) that does not comprise a full-length antibody polypeptide, but that still comprises at least a portion of a full-length antibody polypeptide that is capable of binding to an antigen. Antibody fragments can comprise a cleaved portion of a full length antibody polypeptide, although the term is not limited to such cleaved fragments. Antibody fragments that are useful in the present invention include, for example, Fab fragments, F(ab')2 fragments, scFv (single-chain Fv) fragments, linear antibodies, monospecific or multispecific antibody fragments such as bispecific, trispecific, tetraspecific and multispecific antibodies (e.g., diabodies, triabodies, tetrabodies), monovalent or multivalent antibody fragments such as bivalent, trivalent, tetravalent and multivalent antibodies, minibodies, chelating recombinant antibodies, tribodies or bibodies, intrabodies, nanobodies, small modular immunopharmaceuticals (SMIP), binding-domain immunoglobulin fusion proteins, camelized antibodies, and VHH containing antibodies. Additional examples of antigen-binding antibody fragments are known in the art.

The terms "Complementarity-determining region" or "CDR" refer to short polypeptide sequences within the variable region of both heavy and light chain polypeptides that are primarily responsible for mediating specific antigen recognition. The term "framework region" refers to amino acid sequences within the variable region of both heavy and light chain polypeptides that are not CDR sequences, and are primarily responsible for maintaining correct positioning of the CDR sequences to permit antigen binding. Although the framework regions themselves typically do not directly participate in antigen binding, as is known in the art, certain residues within the framework regions of certain antibodies can directly participate in antigen binding or can affect the ability of one or more amino acids in CDRs to interact with antigen.

Examples of antibodies are anti PCSK-9 mAb (e.g., Alirocumab), anti IL-6 mAb (e.g., Sarilumab), and anti IL-4 mAb (e.g., Dupilumab).

Pharmaceutically acceptable salts of any API described herein are also contemplated for use in a drug or medicament in a drug delivery device. Pharmaceutically acceptable salts are for example acid addition salts and basic salts.

An example drug delivery device may involve a needle-based injection system as described in Table 1 of section 5.2 of ISO 11608-1:2014(E). As described in ISO 11608-1:2014(E), needle-based injection systems may be broadly distinguished into multi-dose container systems and single-dose (with partial or full evacuation) container systems. The container may be a replaceable container or an integrated non-replaceable container.

As further described in ISO 11608-1:2014(E), a multi-dose container system may involve a needle-based injection device with a replaceable container. In such a system, each container holds multiple doses, the size of which may be fixed or variable (pre-set by the user). Another multi-dose container system may involve a needle-based injection device with an integrated non-replaceable container. In such a system, each container holds multiple doses, the size of which may be fixed or variable (pre-set by the user).

As further described in ISO 11608-1:2014(E), a single-dose container system may involve a needle-based injection device with a replaceable container. In one example for such a system, each container holds a single dose, whereby the entire deliverable volume is expelled (full evacuation). In a further example, each container holds a single dose, whereby a portion of the deliverable volume is expelled (partial evacuation). As also described in ISO 11608-1:2014(E), a single-dose container system may involve a needle-based injection device with an integrated non-replaceable container. In one example for such a system, each container holds a single dose, whereby the entire deliverable volume is expelled (full evacuation). In a further example, each container holds a single dose, whereby a portion of the deliverable volume is expelled (partial evacuation).

The terms "axial", "radial", or "circumferential" as used herein may be used with respect to a main longitudinal axis of the device, the cartridge, the housing or the cartridge holder, e.g. the axis which extends through the proximal and distal ends of the cartridge, the cartridge holder or the drug delivery device.

Non-limiting, exemplary embodiments of the disclosure will now be described with reference to the accompanying drawings, in which:
- Figure 1: shows an embodiment of a drug delivery device;
- Figure 2: shows a sectional view on the system of a first embodiment during storage in a packaging;
- Figure 3a: shows a sectional view on the system of the first embodiment fitted to an injection device in 0U position with button not depressed;
- Figure 3b: shows a sectional view on the system of the first embodiment fitted to an injection device in 0U position with button depressed;
- Figure 4: shows a sectional view on the system of a second embodiment fitted to an injection device at the beginning of dispensing a dose, with mechanism dialled out and the button depressed;
- Figure 5: shows an exploded view on the components of the system;
- Figure 6: shows a detail of the chassis of Figure 5; and
- Figure 7: shows a sectional view on the system of a further embodiment during charging in a charging station.

In the figures, identical elements, identically acting elements or elements of the same kind may be provided with the same reference numerals.

In the following, some embodiments will be described with reference to an insulin injection device. The present disclosure is however not limited to such application and may equally well be deployed with injection devices that are configured to eject other medicaments or drug delivery devices in general, preferably pen-type devices and/or injection devices.

Embodiments are provided in relation to injection devices, in particular to variable dose injection devices, which record and/or track data on doses delivered thereby. These data may include the size of the selected dose and/or the size of the actually delivered dose, the time and date of administration, the duration of the administration and the like. Features described herein include the arrangement of sensing elements and power management techniques (e.g. to facilitate small batteries and/or to enable efficient power usage).

Certain embodiments in this document are illustrated with respect to an injection device as described in WO2014033195 or WO2014033197 where an injection button and grip (dose setting member or dose setter) are combined. The injection button may provide the user interface member for initiating and/or performing a dose delivery operation of the drug delivery device. The grip or knob may provide the user interface member for initiating and/or performing a dose setting operation. Both devices are of the dial extension type, i.e. their length increases during dose setting. Other injection devices with the same kinematical behaviour of the dial extension and button during dose setting and dose expelling operational mode are known as, for example, the Kwikpen^{®} device marketed by Eli Lilly and the Novopen^{®} 4 device marketed by Novo Nordisk. An application of the general principles to these devices therefore appears straightforward and further explanations will be omitted. However, the general principles of the present disclosure are not limited to that kinematical behaviour. Certain other embodiments may be conceived for application to an injection device as described in WO2004078239 where there are separate injection button and grip components / dose setting members. Thus, there may be two separate user interface members, one for the dose setting operation and one for the dose delivery operation.

"Distal" is used herein to specify directions, ends or surfaces which are arranged or are to be arranged to face or point towards a dispensing end of the drug delivery device or components thereof and/or point away from, are to be arranged to face away from or face away from the proximal end. On the other hand, "proximal" is used to specify directions, ends or surfaces which are arranged or are to be arranged to face away from or point away from the dispensing end and/or from the distal end of the drug delivery device or components thereof. The distal end may be the end closest to the dispensing and/or furthest away from the proximal end and the proximal end may be the end furthest away from the dispensing end. A proximal surface may face away from the distal end and/or towards the proximal end. A distal surface may face towards the distal end and/or away from the proximal end. The dispensing end may be the needle end where a needle unit is or is to be mounted to the device, for example.

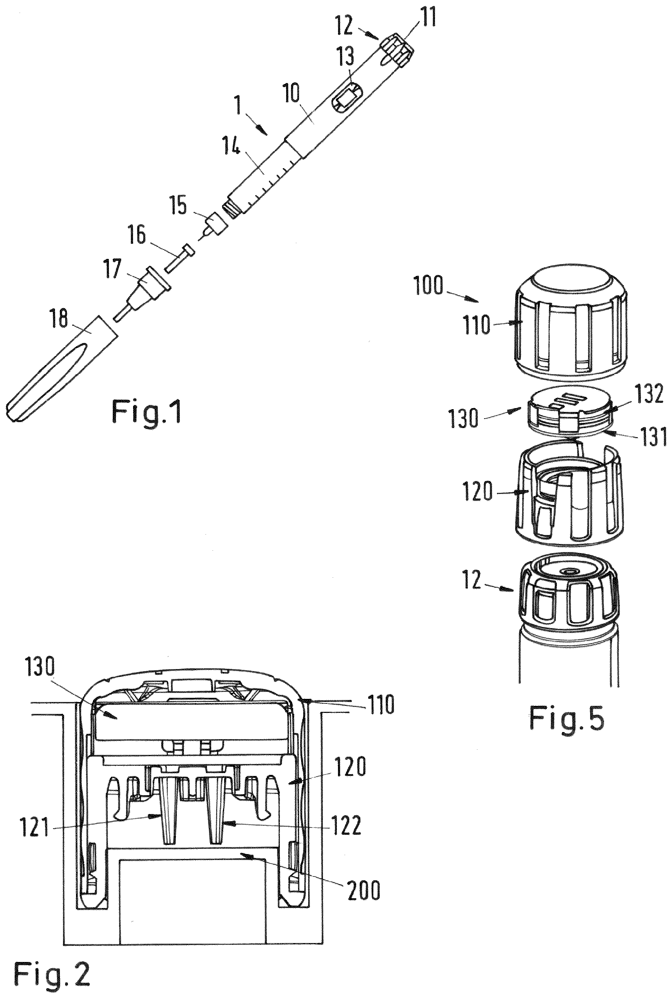

Figure 1 is an exploded view of a medicament delivery device or drug delivery device. In this example, the medicament delivery device is an injection device 1, e.g. a pen-type injector, such as an injection pen as described in WO2014033195 or WO2014033197.

The injection device 1 of Figure 1 is an injection pen that comprises a housing 10 and contains a container 14, e.g. an insulin container, or a receptacle for such a container. The container may contain a drug. A needle 15 can be affixed to the container or the receptacle. The container may be a cartridge and the receptacle may be a cartridge holder. The needle is protected by an inner needle cap 16 and either an outer needle cap 17 or another cap 18. An insulin dose to be ejected from injection device 1 can be set, programmed, or 'dialled in' by turning a dosage knob 12, and a currently programmed or set dose is then displayed via dosage window 13, for instance in multiples of units. The indicia displayed in the window may be provided on a number sleeve or dial sleeve. For example, where the injection device 1 is configured to administer human insulin, the dosage may be displayed in so-called International Units (IU), wherein one IU is the biological equivalent of about 45.5 micrograms of pure crystalline insulin (1/22 mg). Other units may be employed in injection devices for delivering analogue insulin or other medicaments. It should be noted that the selected dose may equally well be displayed differently than as shown in the dosage window 13 in Figure 1.

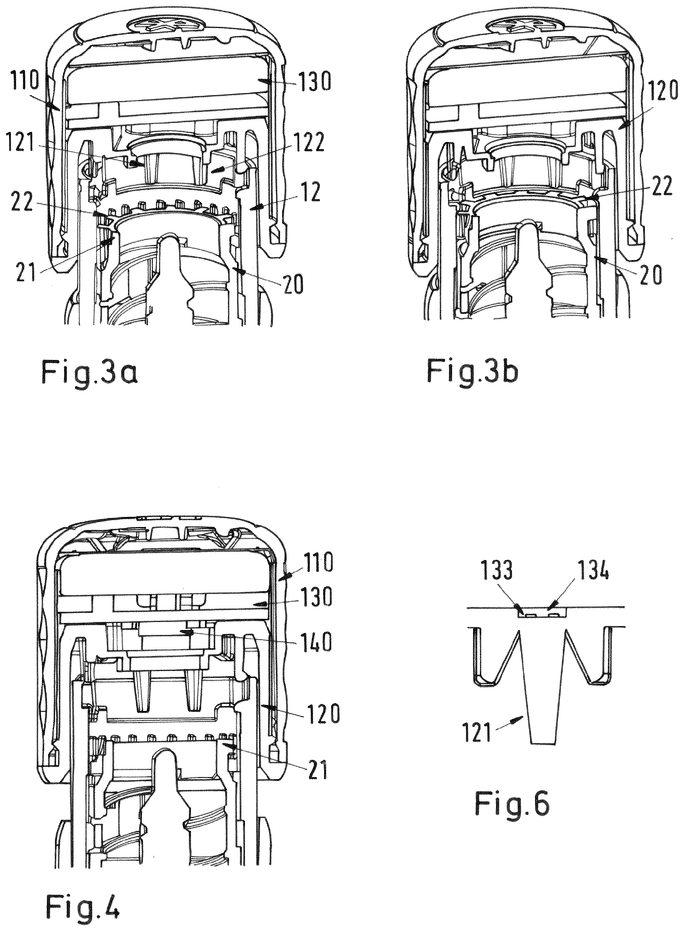

The dosage window 13 may be in the form of an aperture in the housing 10, which permits a user to view a limited portion of a dial sleeve 20 (depicted in Figure 3a) that is configured to move when the dosage knob 12 is turned, to provide a visual indication of a currently set dose. The dosage knob 12 is rotated on a helical path with respect to the housing 10 when setting a dose. In this example, the dosage knob 12 includes one or more formations to facilitate attachment of a data collection device.

The injection device 1 may be configured so that turning the dosage knob 12 causes a mechanical click sound to provide acoustic feedback to a user. In this embodiment, the dosage knob or dose button 12 also acts as an injection button 11. When needle 15 is stuck into a skin portion of a patient, and then dosage knob 12 / injection button 11 is pushed in an axial direction, the insulin dose displayed in display window 13 will be ejected from injection device 1. When the needle 15 of injection device 1 remains for a certain time in the skin portion after the dosage knob 12 is pushed, the dose is injected into the patient's body. Ejection of the insulin dose may also cause a mechanical click sound, which may be different from the sounds produced when rotating the dosage knob 12 during dialing of the dose.

In this embodiment, during delivery of the insulin dose, the dosage knob 12 is returned to its initial position in an axial movement, without rotation, while the dial sleeve 20 is rotated to return to its initial position, e.g. to display a dose of zero units. As noted already, the disclosure is not restricted to insulin but should encompass all drugs in the drug container 14, especially liquid drugs or drug formulations.

Injection device 1 may be used for several injection processes until either the insulin container 14 is empty or the expiration date of the medicament in the injection device 1 (e.g. 28 days after the first use) is reached. In the case of a resuable device, it is possible to replace the insulin container.

Furthermore, before using injection device 1 for the first time, it may be necessary to perform a so-called "prime shot" to remove air from insulin container 14 and needle 15, for instance by selecting two units of insulin and pressing dosage knob 12 while holding injection device 1 with the needle 15 upwards. For simplicity of presentation, in the following, it will be assumed that the selected amounts substantially correspond to the injected doses, so that, for instance the amount of medicament selected from the injection device 1 is equal to the dose received by the user.

As explained above, the dosage knob 12 also functions as an injection button 11 so that the same component is used for dialling/setting the dose and dispensing/delivering the dose.

In the following, an electronic system 100 according to the invention will be described with respect to several different exemplary embodiments. In the depicted exemplary embodiments, the electronic system 100 consitutes a re-usable module which may be releasably attached to the injection device 1, specifically to the button 11 and the dosage knob 12 as indicated in Figure 5. The central longitudinal axis of the injection device 1 is identical with the central axis of the electronic module 100.

The module of the electronic system 100 comprises an outer cap 110, a chassis 120 and a PCB unit 130. The outer cap 110 may be in snap-fit engagement with the chassis 120, thereby axially and rotationally constraining the outer cap 110 and the chassis 120. The electronic system further comprises a communication unit for communicating with another device. When the communication unit is active to perform the manual synchronization or pairing mode, the electronic system has a higher energy consumption. Thus, the communication unit may be switched off when not used.

The chassis 120 is transparent or translucent and may be made from Polycarbonate or the like. The chassis 120 is at least partly received in the outer cap 110 and in turn receives the PCB unit 130. Further, the chassis 120 comprises two light pipes 121, 122 which are axially extending protrusions of the chassis 120 with polished side walls and diffuse end faces forming a sensor side end (upper end in Figure 2) and an encoder side end (lower end in Figure 2). In other words, light may enter or leave the light pipes at the opposite sensor side end and encoder side end but is guiden (reflected) by the side walls. In the example depicted in the Figures, the light pipes 121, 122 are rotationally offset by 45° with respect to each other.

The PCB unit 130 comprises a PCB 131 with a processor and a memory and a coin cell type battery 132. Further, two optical sensor units are provided in the PCB unit 130, e.g. on the PCB 131 facing towards the chassis 120. Each sensor unit comprises a pair of a light source 133, e.g. a LED like an IR transmitter, and an optical sensor 134, e.g. an optical receiver. The sensor units are located directly above the sensor side end of the respective light pipe 121, 122 such that light emitted by the light source 133 passes through the light pipe and exits the light pipe at the encoder side end. If the light is reflected below the encoder side end of the light pipe, it enters back into the light pipe through the encoder side end and is guided to the optical sensor 134 which detects the light signal. However, if the light leaving the encoder side end of the light pipe is absorbed or not reflected back into the light pipe, the optical sensor 134 is not able to detect the light signal.

The button 11 may have apertures permitting the light pipes 121, 122 to pass into the interior of the button 11 and the dosage knob 12. As shown in Figures 3a, 3b and 4, the dial sleeve 20 is provided with an encoder ring 21 in the form of 12 radially extending teeth 22 spaced by 30° from each other. The teeth 22 are made from a light reflecting material or may be coated with a light reflecting material. With the two light pipes 121, 122 positioned 45° apart and the 12 teeth on the dial sleeve encoder ring 21 positioned 30° apart, the two optical sensor units are effectively in anti-phase and for each dose unit that is dispensed, the two channels of the sensor units cross over.

Figures 3a and 3b depict the module 100 fitted onto the injection device 1 in an idle position (Figure 3a), i.e. without any input from a user, and in a position in which a user has pressed button 11 by exerting force on the outer cap 110 (Figure 3b). Comparing Figures 3a and 3b one may see that pressing the button 11 results in shifting the dial grip 12/button 11 together with the attached module 100 towards the dial sleeve 20 and the encoder ring 21, i.e. downwards in the Figures. While the encoder side ends of the light pipes 121, 122 are axially spaced from the encoder ring 21 in the idle position of Figure 3a, the the encoder side ends of the light pipes 121, 122 are directly above the encoder ring 21 and its tteth 22 in the position of Figure 3b.

Figure 2 shows the electronic system 100 in a packaging 200. The packaging 200 comprises a portion located directly beneath the light pipes 121, 122 as shown in Figure 2. At least this portion below the light pipes is light reflective and occludes both light pipes at the encoder side end.

A first operation mode is described with reference to Figures 2 to 3b. In this embodiment, the module 100 is stored in a low-power long-term storage mode when placed in its original packaging 200 immediately after manufacture to reduce battery discharge during long-term storage prior to its first use. In this low-power long-term storage mode, one of the mechanisms for reducing power consumption of the electronic circuit is to disable detection of user inputs. The act of removing the module from its packaging 200 prior to its first use wakes it from this storage mode into a higher-power mode capable of detecting user inputs.

The packaging 200 includes a reflective surface which occludes the end faces of two light pipes 121, 122 connected to two optical sensors 134 in the module. The optical sensors 134 are polled regularly but infrequently during the low-power long-term storage mode (e.g. every 20 seconds). If when the optical sensors 134 are polled both remain occluded, the module 100 remains in the low power long-term storage mode. This polling is only used prior to first use; once it has been detected using this method that the module has been removed from the packaging 200, this mode of regular polling ceases.

When the module 100 is removed from the packaging 200, but before it is fitted to an injection device 1, neither optical sensor 134 is occluded. Likewise, when the module 100 is fitted to an injection device 1 but the dose button 11 is not depressed neither optical sensor 134 is occluded. If when the sensors are polled, the module 100 is in this neither-occluded state, the module will wake.

When the module 100 is fitted to an injection device 1 and the dose button 1 is depressed (e.g. to pair the module 100 to a mobile phone using Bluetooth), a single optical sensor 134 is occluded by one of the teeth 22 of the injection pen 1. If the module 100 is rapidly removed from its packaging 200 and fitted to an injection device 1 and the user holds the dose button 11 in, such that the sensors 134 are not polled in the neither-occluded state described above, when they are polled in this single-occluded state the module 100 will wake.

A second operation mode is described with reference to Figures 3a to 3b. The module may be received in a packaging prior to being mounted onto the injection device 1, but the packaging does not have a light reflective region located below the light pipes 121, 122. In this embodiment, constant low-frequency polling (e.g. 1Hz) of a pair of optical sensors 134 in the module 100 is used in low-power mode to detect when the user has depressed the injection device 1 dose button 11 in order to synchronize and pair the module 100 with a mobile phone using Bluetooth. This constant low-frequency polling is used in low-power mode following first use when the module 100 is inactive, i.e. a dose is not being recorded. It may also be used while the module 100 is in the packaging, but before it is fitted to an injection device 1.

When the module 100 is not attached to an injection device (e.g. it has not yet been removed from its original packaging), the optical sensors are in the neither-occluded state. Likewise, when the module 100 has been fitted to an injection device 1 but the dose button 11 has not been depressed (e.g. the module has not been paired with a mobile phone using Bluetooth), the optical sensors 134 are in the neither-occluded state. If the sensors are polled in this neither-occluded state, the module 100 will not wake.

When the module 100 is fitted to an injection device 1 and the dose button 1 is depressed, one of the two optical sensors 134 is occluded. If the user holds the dose button 11 in this state for a long enough period (e.g. several seconds to initiate pairing) the optical sensors 134 will be polled in this single-occluded state. This will cause the module 100 to wake.

A third operation mode is described with reference to Figures 3a to 3b. In this embodiment, constant moderate-frequency polling (e.g. 200Hz) of a pair of optical sensors 134 in the module 100 is used in low-power mode to detect when the user has begun to dispense a dose using the injection device 1. This constant low-frequency polling is used in low-power mode following first use when the module 100 is inactive, i.e. a dose is not being recorded. It may also be used while the module 100 is in the packaging, but before it is fitted to an injection device 1.

When the dose button 11 is first pressed, one of the two optical sensors 134 will be occluded by a tooth 22 on the dial sleeve 20 and the other will not. As the first unit of drug is dispensed, the dial sleeve 20 will rotate such that the optical sensor 134 which was initially occluded ceases to be so, and the optical sensor 134 which was not initially occluded becomes so.

The moderate-frequency polling of the optical sensors will allow this change of occlusion state to be detected, and the module 100 will wake and immediately record the dose being dispensed.

A fourth operation mode is describe with reference to Figure 4 which shows a second embodiment of the module 100 further comprising a motion sensor 140 located e.g. centrally on the PCB unit 130. In this embodiment, the motion sensor 140 is used while in low-power mode to detect whether the module 100 is stationary. If no movement is detected, the module 100 is assumed to be in storage or idle, with no user interaction occurring, the optical sensors 134 are not polled and the module 100 does not wake. If movement is detected, it is assumed that the user may be interacting with the module 100, the module 100 partially wakes into the low-frequency polling mode (second operation mode) or into the moderate-frequency polling mode (third operation mode) described above. This reduces the amount of power being drawn when the module is sitting stationary and increases battery life relative to the second or third operation mode.

The general working principle of the module 100, its design and interaction with an injection device 1 may be similar as disclosed in WO2021/191326A1 or in WO 2021/116387 A1 to which reference is made.

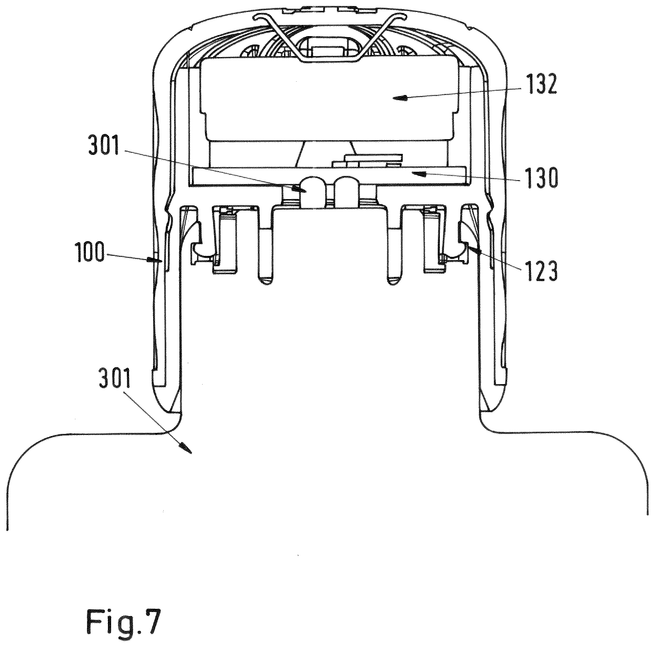

A still further embodiment of the electrical system 100 is depicted in Figure 7 wherein the battery 132 is a rechargeable battery. The module 100 is depicted removed from a drug delivery device and put on a charging station 300 having two pins 301 contacting and connected to conductive pads provided on the PCBA 130 of the module. The charging station may be provided with a substantially cylindrical or conical socket having pins 301 on its top. Cap 110 and chassis 120 encase the socket to retain the module 100 on the charging station 300. Clip features 123 of the chassis 120 may engage corresponding retention features of the charging station 300 to hold the module 100 on the charging station 300 in the correct axial and/or rotational position. There may be a gasket seal between the chassis 120 and the PCB 130 around the charging pin locations. An optional motion sensor 140 (not shown in Figure 7), e.g. an accelerometer, may be provided in module 100 for waking the system from a sleeping state in which the light sources 133 are not activated.

### Reference Numerals

- 1: device
- 10: housing
- 11: injection button
- 12: dosage knob
- 13: dosage window
- 14: container/container receptacle
- 15: needle
- 16: inner needle cap
- 17: outer needle cap
- 18: cap
- 20: dose dial sleeve
- 21: encoder
- 22: teeth
- 100: electrical system
- 110: cap
- 120: chassis
- 121: light pipe
- 122: light pipe
- 123: clip feature
- 130: PCB unit
- 131: PCB (processor and memory)
- 132: battery
- 133: light source (IR LED)
- 134: optical sensor
- 140: motion senseor
- 200: packaging
- 300: charging station
- 301: pin

## Claims

1. An electronic system for use with a drug delivery device (1) for recording doses that are delivered from the drug delivery device (1), the electronic system comprising:
an electrical power supply (132),
a memory,
a processor (131) configured to control operation of the electronic system and coupled to the electrical power supply (132) and to the memory, and
a first light source (133), a second light source, a first optical sensor (134) and a second optical sensor which are in communication with the processor (131), wherein the optical sensors (134) are suitable for detecting a movement of an encoder (21) of the drug delivery device in an anti-phase arrangement which movement is indicative of doses that are dialed and/or delivered from the drug delivery device (1),
the electronic system having a first low-power-consumption state and at least one further state having a higher power consumption compared with the first state,
**characterized in that** the processor (131) is configured to regularly poll both optical sensors (134) in the first low-power-consumption state, to maintain the first low-power-consumption state if the response of both optical sensors (134) is identical to each other and identical to the preceding response, and to switch into the at least one further state if the responses of the optical sensors (134) are different from each other or are different from the preceding response.

2. The electronic system according to claim 1, wherein the first light source (133) and the first optical sensor (134) are arranged next to each other and facing towards a sensor side end of a first light pipe (121), wherein the second light source and the second optical sensor are arranged next to each other and facing towards a sensor side end of a second light pipe (122), and wherein the light pipes (121, 122) each have an opposite encoder side end which if occluded by a light reflective material (200) result in light emitted from the respective first or second light source (133) to be detected by the respective first or second optical sensor (134).

3. The electronic system according to claim 1 or 2, further comprising at least one removable occluding element (200) positioned in the respective light path between the light sources (133) and the respective optical sensors (134).

4. The electronic system according to claim 3, wherein the removable occluding element (200) comprises a reflective surface for occluding the optical sensors (134).

5. The electronic system according to any one of the preceding claims, wherein the processor (131) is configured to regularly poll the optical sensors (134) in the first low-power-consumption state, for example every 10 to 40 seconds, ideally every 15 to 25 seconds, e.g. every 20 seconds, and wherein the processor (131) is configured to regularly poll the optical sensors (134) in the at least one further state with a higher frequency than in the first low-power-consumption state.

6. The electronic system according to claim 5, wherein the processor (131) is configured to maintain the first low-power-consumption state if the response of both optical sensors (134) is that light emitted from both light sources (133) is detected by the respective first and second optical sensors (134).

7. The electronic system according to any one of claims 1 to 4 further comprising a communication unit for communicating with another device, wherein the processor (131) is configured to regularly poll the optical sensors (134) in the first low-power-consumption state, for example every 0.5 to 2 seconds, e.g. with a frequency of 1 Hz, and wherein the processor (131) is configured to regularly poll the optical sensors (134) in the at least one further state with a higher frequency than in the first low-power-consumption state and/or to switch on the communication unit.

8. The electronic system according to claim 7, wherein the processor (131) is configured to maintain the first low-power-consumption state if the response of both optical sensors (134) is that light beams emitted from both light sources (133) are detected by neither of both optical sensors (134) and to switch into the at least one further state if the responses of the optical sensors (134) are different from each other and are different from the preceding response.

9. The electronic system according to any one of claims 1 to 4, wherein the processor (133) is configured to regularly poll the optical sensors (134) in the first low-power-consumption state with a frequency of, for example 100 Hz to 300 Hz, e.g. 200 Hz, and wherein the processor (131) is configured to regularly poll the optical sensors (134) in the at least one further state with a higher frequency than in the first low-power-consumption state.

10. The electronic system according to any one of the preceding claims further having a sleeping state in which the light sources (133) are not activated, wherein the electronic system further comprises at least one motion sensor (140) suitable for detecting movement of the electronic system, wherein the processor (131) is configured to maintain the sleeping state if no movement is detected by the at least one motion sensor (140) and to switch into the first low-power-consumption state or into the at least one further state if a movement is detected by the at least one motion sensor (140).

11. The electronic system according to any one of the preceding claims, wherein the at least one further state is a dose recording state in which, when the electronic system is attached to or integrated into a drug delivery device, the light sources (133) and the optical sensors (134) are used to detect dose dialing and/or dose delivering, and wherein the processor (131) is configured to process and/or record the responses of the optical sensors (134) in the dose recording state.

12. The electronic system according to any one of the preceding claims further comprising an outer cap (110) with a cenral axis, a chassis (120) which is at least partially retained within the cap (110) and a PCB unit (130) comprising the memory and the processor (131), wherein the PCB unit (130) and the electrical power supply (132) are retained in the cap (110) and the chassis (120), and wherein the light sources (133) and the optical sensors (134) are arranged on a circular region about the central axis, with the first light source (133) and the first optical sensor (134) being angularly offset from the second light source and the second optical sensor.

13. The electronic system according to claims 2 and 12, wherein the chassis (120) is at least partially transparent and comprises the light pipes (121, 122) which are arranged between 30° and 60°, e.g. 45°, apart from each other.

14. A drug delivery device (1) comprising an electronic system according to any one of the preceding claims and a dose setting and drive mechanism which is configured to perform a dose dialing operation for selecting a dose to be delivered by the drug delivery device and a dose delivery operation for delivering the set dose, the dose setting and drive mechanism comprising an encoder (21) rotatable at least in the dose delivery operation and/or in the dose setting operation, wherein the encoder (21) comprises a ring of 12 light reflecting teeth (22) spaced by 30° from each other, and wherein the electronic system (100) is arranged such that depending on the rotational position of the encoder (21) light emitted by one of the light sources (133) is reflected by one of the teeth (22) and detected by the respective optical sensor (134), while light emitted by the other of the light sources is not reflected by one of the teeth.

15. The drug delivery device according to claim 14, further comprising a cartridge containing a medicament.

## Patentansprüche

1. Elektronisches System zur Verwendung mit einer Medikamenten-Verabreichungsvorrichtung (1) zum Aufzeichnen von Dosen, die von der Medikamenten-Verabreichungsvorrichtung (1) verabreicht werden, das elektronische System umfassend:
eine elektrische Stromversorgung (132),
einen Speicher,
einen Prozessor (131), der so konfiguriert ist, dass er den Betrieb des elektronischen Systems steuert, und der mit der elektrischen Stromversorgung (132) und dem Speicher gekoppelt ist, und
eine erste Lichtquelle (133), eine zweite Lichtquelle, einen ersten optischen Sensor (134) und einen zweiten optischen Sensor, die mit dem Prozessor (131) kommunizieren, wobei die optischen Sensoren (134) dazu geeignet sind, eine Bewegung eines Encoders (21) der Medikamenten-Verabreichungsvorrichtung in einer gegenphasigen Anordnung zu erfassen, wobei diese Bewegung Dosen anzeigt, die von der Medikamenten-Verabreichungsvorrichtung (1) angewählt und/oder verabreicht werden,
wobei das elektronische System einen ersten Zustand mit geringem Stromverbrauch und mindestens einen weiteren Zustand mit einem im Vergleich zum ersten Zustand höheren Stromverbrauch aufweist,
**dadurch gekennzeichnet, dass** der Prozessor (131) so konfiguriert ist, dass er in dem ersten Zustand mit geringem Stromverbrauch regelmäßig beide optischen Sensoren (134) abfragt, um den ersten Zustand mit geringem Stromverbrauch aufrechtzuerhalten, wenn die Reaktion beider optischen Sensoren (134) identisch miteinander und identisch mit der vorhergehenden Reaktion ist, und in den mindestens einen weiteren Zustand zu wechseln, wenn die Reaktionen der optischen Sensoren (134) voneinander abweichen oder von der vorhergehenden Reaktion abweichen.

2. Elektronisches System nach Anspruch 1, wobei die erste Lichtquelle (133) und der erste optische Sensor (134) nebeneinander angeordnet sind und zu einem sensorseitigen Ende eines ersten Lichtleiters (121) hin ausgerichtet sind, wobei die zweite Lichtquelle und der zweite optische Sensor nebeneinander angeordnet sind und zu einem sensorseitigen Ende eines zweiten Lichtleiters (122) hin ausgerichtet sind, und wobei die Lichtleiter (121, 122) jeweils ein gegenüberliegendes Encoderseitiges Ende aufweisen, das, wenn es durch ein lichtreflektierendes Material (200) abgedeckt wird, dazu führt, dass von der jeweiligen ersten oder zweiten Lichtquelle (133) emittiertes Licht von dem jeweiligen ersten oder zweiten optischen Sensor (134) erfasst wird.

3. Elektronisches System nach Anspruch 1 oder 2, das ferner mindestens ein abnehmbares Abdeckelement (200) umfasst, das in dem jeweiligen Lichtweg zwischen den Lichtquellen (133) und den jeweiligen optischen Sensoren (134) positioniert ist.

4. Elektronisches System nach Anspruch 3, wobei das abnehmbare Abdeckelement (200) eine reflektierende Oberfläche zum Abdecken der optischen Sensoren (134) umfasst.

5. Elektronisches System nach einem der vorhergehenden Ansprüche, wobei der Prozessor (131) so konfiguriert ist, dass er die optischen Sensoren (134) in dem ersten Zustand mit geringem Stromverbrauch regelmäßig abfragt, beispielsweise alle 10 bis 40 Sekunden, idealerweise alle 15 bis 25 Sekunden, z. B. alle 20 Sekunden, und wobei der Prozessor (131) so konfiguriert ist, dass er die optischen Sensoren (134) in dem mindestens einen weiteren Zustand regelmäßig mit einer höheren Frequenz als in dem ersten Zustand mit geringem Stromverbrauch abfragt.

6. Elektronisches System nach Anspruch 5, wobei der Prozessor (131) so konfiguriert ist, dass er den ersten Zustand mit geringem Stromverbrauch aufrechterhält, wenn die Reaktion beider optischen Sensoren (134) darin besteht, dass das von beiden Lichtquellen (133) emittierte Licht von dem jeweiligen ersten und zweiten optischen Sensor (134) erfasst wird.

7. Elektronisches System nach einem der Ansprüche 1 bis 4, das ferner eine Kommunikationseinheit zum Kommunizieren mit einer anderen Vorrichtung umfasst, wobei der Prozessor (131) so konfiguriert ist, dass er die optischen Sensoren (134) in dem ersten Zustand mit geringem Stromverbrauch regelmäßig abfragt, beispielsweise alle 0,5 bis 2 Sekunden, z. B. mit einer Frequenz von 1 Hz, und wobei der Prozessor (131) so konfiguriert ist, dass er die optischen Sensoren (134) in dem mindestens einen weiteren Zustand regelmäßig mit einer höheren Frequenz als in dem ersten Zustand mit geringem Stromverbrauch abfragt und/oder die Kommunikationseinheit einschaltet.

8. Elektronisches System nach Anspruch 7, wobei der Prozessor (131) so konfiguriert ist, dass er den ersten Zustand mit geringem Stromverbrauch aufrechterhält, wenn die Reaktion beider optischen Sensoren (134) darin besteht, dass von beiden Lichtquellen (133) emittierte Lichtstrahlen von keinem der beiden optischen Sensoren (134) erfasst werden, und in den mindestens einen weiteren Zustand wechselt, wenn die Reaktionen der optischen Sensoren (134) voneinander abweichen und von der vorherigen Reaktion abweichen.

9. Elektronisches System nach einem der Ansprüche 1 bis 4, wobei der Prozessor (133) so konfiguriert ist, dass er die optischen Sensoren (134) in dem ersten Zustand mit geringem Stromverbrauch regelmäßig mit einer Frequenz von beispielsweise 100 Hz bis 300 Hz, z. B. 200 Hz, abfragt, und wobei der Prozessor (131) so konfiguriert ist, dass er die optischen Sensoren (134) in dem mindestens einen weiteren Zustand regelmäßig mit einer höheren Frequenz als in dem ersten Zustand mit geringem Stromverbrauch abfragt.

10. Elektronisches System nach einem der vorhergehenden Ansprüche, das ferner einen Ruhezustand aufweist, in dem die Lichtquellen (133) nicht aktiviert sind, wobei das elektronische System ferner mindestens einen Bewegungssensor (140) umfasst, der zum Erfassen von Bewegungen des elektronischen Systems geeignet ist, wobei der Prozessor (131) so konfiguriert ist, dass er den Ruhezustand aufrechterhält, wenn keine Bewegung durch den mindestens einen Bewegungssensor (140) erfasst wird, und in den ersten Zustand mit geringem Stromverbrauch oder in den mindestens einen weiteren Zustand wechselt, wenn eine Bewegung durch den mindestens einen Bewegungssensor (140) erfasst wird.

11. Elektronisches System nach einem der vorhergehenden Ansprüche, wobei der mindestens eine weitere Zustand ein Dosisaufzeichnungszustand ist, in dem, wenn das elektronische System an einer Medikamenten-Verabreichungsvorrichtung angebracht oder in diese integriert ist, die Lichtquellen (133) und die optischen Sensoren (134) verwendet werden, um die Dosisanwahl und/oder die Dosisverabreichung zu erfassen, und wobei der Prozessor (131) so konfiguriert ist, dass er die Reaktionen der optischen Sensoren (134) in dem Dosisaufzeichnungszustand verarbeitet und/oder aufzeichnet.

12. Elektronisches System nach einem der vorhergehenden Ansprüche, das ferner eine Außenkappe (110) mit einer Mittelachse, ein Gehäuse (120), das zumindest teilweise in der Kappe (110) gehalten wird, und eine Leiterplatteneinheit (130) umfasst, die den Speicher und den Prozessor (131) umfasst, wobei die Leiterplatteneinheit (130) und die Stromversorgung (132) in der Kappe (110) und dem Gehäuse (120) gehalten werden, und wobei die Lichtquellen (133) und die optischen Sensoren (134) in einem kreisförmigen Bereich um die Mittelachse angeordnet sind, wobei die erste Lichtquelle (133) und der erste optische Sensor (134) gegenüber der zweiten Lichtquelle und dem zweiten optischen Sensor winklig versetzt sind.

13. Elektronisches System nach den Ansprüchen 2 und 12, wobei das Gehäuse (120) zumindest teilweise transparent ist und die Lichtleiter (121, 122) umfasst, die in einem Winkel von 30° bis 60°, beispielsweise bei 45°, zueinander angeordnet sind.

14. Medikamenten-Verabreichungsvorrichtung (1), die ein elektronisches System nach einem der vorhergehenden Ansprüche und einen Dosiseinstell- und Dosisantriebsmechanismus umfasst, der so konfiguriert ist, dass er einen Dosisanwählvorgang zum Auswählen einer von der Medikamenten-Verabreichungsvorrichtung zu verabreichenden Dosis und einen Dosisverabreichungsvorgang zum Verabreichen der eingestellten Dosis durchführt, wobei der Dosiseinstell- und Dosisantriebsmechanismus einen Encoder (21) umfasst, der zumindest während des Dosisverabreichungsvorgangs und/oder des Dosiseinstellvorgangs drehbar ist, wobei der Encoder (21) einen Ring aus 12 lichtreflektierenden Zähnen (22) umfasst, die in einem Abstand von 30° voneinander beabstandet sind, und wobei das elektronische System (100) so angeordnet ist, dass in Abhängigkeit von der Drehposition des Encoders (21) von einer der Lichtquellen (133) emittiertes Licht von einem der Zähne (22) reflektiert und von dem jeweiligen optischen Sensor (134) erfasst wird, während von der anderen Lichtquelle emittiertes Licht nicht von einem der Zähne reflektiert wird.

15. Medikamenten-Verabreichungsvorrichtung nach Anspruch 14, ferner umfassend eine Kartusche, die ein Medikament enthält.

## Revendications

1. Système électronique destiné à être utilisé avec un dispositif d'administration de médicament (1) pour enregistrer des doses qui sont administrées à partir du dispositif d'administration de médicament (1), le système électronique comprenant :
une alimentation électrique (132),
une mémoire,
un processeur (131) configuré pour commander le fonctionnement du système électronique et couplé à l'alimentation électrique (132) et à la mémoire, et
une première source de lumière (133), une seconde source de lumière, un premier capteur optique (134) et un second capteur optique qui sont en communication avec le processeur (131), les capteurs optiques (134) étant appropriés pour détecter un mouvement d'un codeur (21) du dispositif d'administration de médicament dans un agencement en opposition de phase, lequel mouvement est indicatif de doses qui sont composées et/ou administrées à partir du dispositif d'administration de médicament (1),
le système électronique ayant un premier état de faible consommation d'énergie et au moins un autre état ayant une consommation d'énergie supérieure par rapport au premier état,
**caractérisé en ce que** le processeur (131) est configuré pour interroger régulièrement les deux capteurs optiques (134) dans le premier état de faible consommation d'énergie, pour maintenir le premier état de faible consommation d'énergie si la réponse des deux capteurs optiques (134) est identique l'une à l'autre et identique à la réponse précédente, et pour commuter dans l'au moins un autre état si les réponses des capteurs optiques (134) sont différentes l'une de l'autre ou sont différentes de la réponse précédente.

2. Système électronique selon la revendication 1, la première source de lumière (133) et le premier capteur optique (134) étant agencés l'un à côté de l'autre et faisant face vers une extrémité côté capteur d'un premier conduit de lumière (121), la seconde source de lumière et le second capteur optique étant agencés l'un à côté de l'autre et faisant face vers une extrémité côté capteur d'un second conduit de lumière (122), et les conduits de lumière (121, 122) ayant chacun une extrémité côté codeur opposée qui si occultée par un matériau réfléchissant la lumière (200) résulte en ce que la lumière émise par la première ou seconde source de lumière respective (133) soit détectée par le premier ou second capteur optique respectif (134).

3. Système électronique selon la revendication 1 ou 2, comprenant en outre au moins un élément d'occlusion amovible (200) positionné dans le trajet lumineux respectif entre les sources de lumière (133) et les capteurs optiques respectifs (134).

4. Système électronique selon la revendication 3, l'élément d'occlusion amovible (200) comprenant une surface réfléchissante pour occlure les capteurs optiques (134).

5. Système électronique selon l'une quelconque des revendications précédentes, le processeur (131) étant configuré pour interroger régulièrement les capteurs optiques (134) dans le premier état de faible consommation d'énergie, par exemple toutes les 10 à 40 secondes, idéalement toutes les 15 à 25 secondes, par exemple toutes les 20 secondes, et le processeur (131) étant configuré pour interroger régulièrement les capteurs optiques (134) dans l'au moins un autre état avec une fréquence plus élevée que dans le premier état de faible consommation d'énergie.

6. Système électronique selon la revendication 5, le processeur (131) étant configuré pour maintenir le premier état de faible consommation d'énergie si la réponse des deux capteurs optiques (134) est que la lumière émise par les deux sources de lumière (133) est détectée par les premier et second capteurs optiques respectifs (134).

7. Système électronique selon l'une quelconque des revendications 1 à 4, comprenant en outre une unité de communication pour communiquer avec un autre dispositif, le processeur (131) étant configuré pour interroger régulièrement les capteurs optiques (134) dans le premier état de faible consommation d'énergie, par exemple toutes les 0,5 à 2 secondes, par exemple avec une fréquence de 1 Hz, et le processeur (131) étant configuré pour interroger régulièrement les capteurs optiques (134) dans l'au moins un autre état avec une fréquence plus élevée que dans le premier état de faible consommation d'énergie et/ou pour commuter sur l'unité de communication.

8. Système électronique selon la revendication 7, le processeur (131) étant configuré pour maintenir le premier état de faible consommation d'énergie si la réponse des deux capteurs optiques (134) est que des faisceaux lumineux émis par les deux sources de lumière (133) sont détectés par aucun des deux capteurs optiques (134) et pour commuter dans l'au moins un autre état si les réponses des capteurs optiques (134) sont différentes l'une de l'autre et sont différentes de la réponse précédente.

9. Système électronique selon l'une quelconque des revendications 1 à 4, le processeur (133) étant configuré pour interroger régulièrement les capteurs optiques (134) dans le premier état de faible consommation d'énergie avec une fréquence de, par exemple 100 Hz à 300 Hz, par exemple 200 Hz, et le processeur (131) étant configuré pour interroger régulièrement les capteurs optiques (134) dans l'au moins un autre état avec une fréquence plus élevée que dans le premier état de faible consommation d'énergie.

10. Système électronique selon l'une quelconque des revendications précédentes ayant en outre un état de sommeil dans lequel les sources de lumière (133) ne sont pas activées, le système électronique comprenant en outre au moins un capteur de mouvement (140) approprié pour détecter un mouvement du système électronique, le processeur (131) étant configuré pour maintenir l'état de sommeil si aucun mouvement n'est détecté par l'au moins un capteur de mouvement (140) et pour commuter dans le premier état de faible consommation d'énergie ou dans l'au moins un autre état si un mouvement est détecté par l'au moins un capteur de mouvement (140).

11. Système électronique selon l'une quelconque des revendications précédentes, l'au moins un autre état étant un état d'enregistrement de dose dans lequel, lorsque le système électronique est attaché à ou intégré dans un dispositif d'administration de médicament, les sources de lumière (133) et les capteurs optiques (134) sont utilisés pour détecter la composition de dose et/ou l'administration de dose, et le processeur (131) étant configuré pour traiter et/ou enregistrer les réponses des capteurs optiques (134) dans l'état d'enregistrement de dose.

12. Système électronique selon l'une quelconque des revendications précédentes comprenant en outre un capuchon extérieur (110) avec un axe central, un châssis (120) qui est au moins partiellement retenu à l'intérieur du capuchon (110) et une unité PCB (130) comprenant la mémoire et le processeur (131), l'unité PCB (130) et l'alimentation électrique (132) étant retenues dans le capuchon (110) et le châssis (120), et les sources de lumière (133) et les capteurs optiques (134) étant agencés sur une région circulaire autour de l'axe central, la première source de lumière (133) et le premier capteur optique (134) étant décalés angulairement par rapport à la seconde source de lumière et au second capteur optique.

13. Système électronique selon les revendications 2 et 12, le châssis (120) étant au moins partiellement transparent et comprenant les conduits de lumière (121, 122) qui sont agencés entre 30° et 60°, par exemple 45°, espacés l'un de l'autre.

14. Dispositif d'administration de médicament (1) comprenant un système électronique selon l'une quelconque des revendications précédentes et un mécanisme de réglage et d'entraînement de dose qui est configuré pour effectuer une opération de composition de dose pour sélectionner une dose à administrer par le dispositif d'administration de médicament et une opération d'administration de dose pour administrer la dose réglée, le mécanisme de réglage et d'entraînement de dose comprenant un codeur (21) rotatif au moins dans l'opération d'administration de dose et/ou dans l'opération de réglage de dose, le codeur (21) comprenant un anneau de 12 dents réfléchissant la lumière (22) espacées de 30° les unes des autres, et le système électronique (100) étant agencé de telle sorte qu'en fonction de la position de rotation du codeur (21) la lumière émise par l'une des sources de lumière (133) est réfléchie par l'une des dents (22) et détectée par le capteur optique respectif (134), tandis que la lumière émise par l'autre des sources de lumière n'est pas réfléchie par l'une des dents.

15. Dispositif d'administration de médicament selon la revendication 14, comprenant en outre une cartouche contenant un médicament.
